# EUROPÄISCHE PATENTSCHRIFT

(11) **EP 2 343 091 B1**
(45) Veröffentlichungstag und Bekanntmachung des Hinweises auf die Patenterteilung: **14.05.2014**
(21) Anmeldenummer: 10075014.0
(22) Anmeldetag: 08.01.2010
(51) Int. Cl.: A61M 1/10

(54) **Fluidpumpe mit einer Fördereinrichtung mit steuerbarer Volumenänderung**
Fluid pump with a transport device with controllable volume alteration
Pompe à fluide dotée d'un dispositif de transport équipé d'une variation de volume commandable

(43) Veröffentlichungstag der Anmeldung: 13.07.2011
(73) Patentinhaber: ECP Entwicklungsgesellschaft mbH, 12247 Berlin (DE)
(72) Erfinder: Töllner, Thomas, 12047 Berlin (DE)
(74) Vertreter: Pfenning, Meinig & Partner GbR

(56) Entgegenhaltungen:
- EP-A1- 2 016 961
- EP-A1- 2 047 872
- WO-A1-96/32145
- DE-A1- 2 701 171
- FR-A- 1 448 822
- US-A- 3 720 200
- US-A- 3 939 820
- US-A- 4 902 272
- US-A1- 2008 281 147
- US-B1- 6 210 318

## Beschreibung

Die vorliegende Erfindung liegt auf dem Gebiet des Maschinenbaus, insbesondere der Mikromechanik, und bezieht sich auf Fluidpumpen.

Derartige Fluidpumpen, besonders in kleiner Bauart, werden dort eingesetzt, wo entweder kleine Flüssigkeits- oder Gasmengen transportiert werden müssen, die vorzugsweise genau dosiert werden, oder an schwer zugänglichen Stellen, in die größere Pumpen schlecht eingebracht werden können.

Ein großer Anwendungsbereich solcher Pumpen ergibt sich beispielsweise in der Medizintechnik, wo Pumpen entweder als Dosierpumpen, beispielsweise für die Dosierung von Medikamenten, oder als Förderpumpen für körpereigene Flüssigkeiten wie beispielsweise Blut eingesetzt werden.

Es sind verschiedene Bauarten für Blutpumpen bekannt geworden, die zur Unterstützung oder zum Ersatz der Herzfunktion eingesetzt werden und die unter minimalinvasiven Bedingungen durch ein körpereigenes Blutgefäß beispielsweise in eine Herzkammer eingebracht oder auch in einem Blutgefäß betrieben werden können.

Solche Pumpen haben Baugrößen, die entweder im Durchmesser dem zur Verfügung stehenden Innendurchmesser eines Blutgefäßes entsprechen oder die zumindest für den Transport auf eine solche Größe kollabiert werden und später, wenn sie beispielsweise in eine Herzkammer eingebracht worden sind, dort expandiert werden können.

Eine Vielzahl solcher Pumpen weist rotierende Förderelemente in Form von Rotoren mit Förderschaufeln auf, die schnell rotieren und damit Blut fördern, wie z.B. in EP 2 047 872 offenbart.

Es sind jedoch auch Pumpen bekannt geworden, die ohne ein rotierendes Förderelement auskommen, wie beispielsweise in der WO 002008/113785 offenbart. Dort ist innerhalb des Fluidraums eines Pumpengehäuses ein expandierbares und kollabierbares Förderelement beschrieben. Es werden wechselweise Einström- und Ausströmklappen betrieben, die je nach der Phase des Einsaugens und der Verdrängung von Fluid im Fluidraum geschlossen bzw. geöffnet sind, um einen gerichteten Volumenstrom zu erzeugen. Ähnliche Pumpen sind in US 6,210,318 und EP 2 016 961 offenbart.

Die vorliegende Erfindung hat sich zur Aufgabe gestellt, eine Fluidpumpe mit einem Fluidraum, einem Einströmkanal und einem Ausströmkanal sowie einer Fördereinrichtung mit einem bezüglich seines Volumens steuerbaren Element in Konstruktion und Betrieb zu vereinfachen und besonders zuverlässig zu gestalten.

Diese Aufgabe wird mit den Merkmalen der Erfindung gemäß dem Patentanspruch 1 gelöst.

Es sind ein oder mehrere Sperrelemente vorgesehen, die bezüglich ihres Volumens steuerbar sind und jeweils im Einströmkanal und/oder im Ausströmkanal des Gehäuses liegen. Im Zuge der Volumensteuerung ist bei jedem Sperrelement jeweils mindestens ein erstes Volumen und ein zweites Volumen einstellbar, wobei beim ersten Volumen des Sperrelementes der entsprechende Kanal wenigstens überwiegend, insbesondere ganz, freigegeben wird und der entsprechende Kanal bei einem zweiten eingestellten Volumen des Sperrelementes wenigstens überwiegend, insbesondere vollständig gesperrt wird. Maßgeblich ist dabei, dass der Strömungswiderstand für das Fluid bei dem ersten Volumen des Sperrelementes wesentlich geringer ist, als wenn dieses das zweite Volumen annimmt.

Dies kann dadurch realisiert sein, dass das erste Volumen geringer ist als das zweite Volumen und dass das Sperrelement durch die Ausdehnung auf das zweite Volumen den Einströmkanal/Ausströmkanal möglichst weitgehend sperrt.

Es kann jedoch beispielsweise auch vorgesehen sein, dass durch Vergrößerung des Volumens des Sperrelements der Einströmkanal aufgeweitet und damit entsperrt wird und dass bei einer Volumenverringerung des Sperrelementes eine Sperrung des Kanals eintritt.

Das Sperrelement kann die Form eines einfachen rotationssymmetrischen ballonartigen, kugelförmigne oder zylindrischen Körpers aufweisen, der eine Durchgangsöffnung bei vergrößertem Volumen versperrt, jedoch kann das Sperrelement auch die Form eines Torus aufweisen, der einen Kanal in seinem Inneren frei lässt und diesen bei einer Volumenverringerung des Torus durch Expansion radial nach innen verschließt.

Zudem sieht die Erfindung vor, dass durch die Fördereinrichtung der Teil des in dem Fluidraum durch das erste Fluid ausfüllbaren Volumens durch eine steuerbare Volumenänderung verändert werden kann. Auf diese Weise kann das Sperrelement zur Steuerung des Ein-/Ausströmkanals verwendet werden, und durch entsprechende weitere Expansion nach Sperrung eines Kanals kann dann gezielt das Fluid durch den jeweils verbleibenden offenen Kanal, insbesondere den Ausströmkanal, ausgepresst werden.

Eine vorteilhafte Ausgestaltung der Erfindung sieht vor, dass das/die volumensteuerbare(n) Element(e) jeweils einen mit einem zweiten Fluid füllbaren Hohlraum aufweist/aufweisen. Diese Konstruktionsform erlaubt die Steuerung des/der Sperrelement(e) und eines eventuell zusätzlichen Verdrängungselements der Fördereinrichtung durch Füllen mit einem zweiten Fluid. Die Steuerung kann dann beispielsweise mittels einer einzigen Druckquelle für das zweite Fluid und entsprechender Ventilsteuerung erfolgen. Es müssen keine Ventile gesteuert werden, die auf anderen Funktionsmechanismen beruhen, wie beispielsweise mechanischer motorischer Betätigung oder selbsttätigem Öffnen und Schließen von Rückschlagventilen. Dadurch ist eine erhöhte Zuverlässigkeit und verbesserte Steuermöglichkeit gegeben.

Grundsätzlich können die bezüglich des Volumens steuerbaren Elemente eine flexible Außenwand aufweisen, um durch Volumenänderung straff aufpumpbar bzw. kollabierbar zu sein. Besonders vorteilhaft ist es jedoch, wenn die Außenwand elastisch ist, so dass das Kollabieren durch die elastischen Kräfte der Außenhaut bei Druckreduzierung sehr zuverlässig und beschleunigt geschieht. Ein solches steuerbares Element passt sich auch bei Volumenzunahme der Öffnung bzw. dem Kanal, den es verschließen soll, flexibel an, so dass ein besonders guter und zuverlässiger Verschluss gegeben ist.

Besonders vorteilhaft kann die Erfindung dadurch ausgestaltet werden, dass wenigstens ein Sperrelement bezüglich seines Volumens auf ein drittes Volumen ausgedehnt werden kann, bei dem es über den beim ersten und zweiten Volumen eingenommenen Raum hinaus wenigstens einen Teil des Fluidraums ausfüllt.

Das entsprechende Sperrelement kann dann zusätzlich zu seiner Sperr- und Entsperrfunktion durch Volumenvergrößerung auf ein drittes Volumen zusätzlich als Verdrängungselement eingesetzt werden und einen Teil des Volumens im Fluidraum zur Verdrängung des darin befindlichen Fluids einnehmen. Dies hat den Vorteil, dass keine zusätzlichen Verdrängungselemente eingesetzt angesteuert und positioniert werden müssen.

Wichtig ist beim Betrieb eines solchen Sperrelements, dass bei der Volumenänderung zunächst der Ein-/Ausströmkanal verschlossen und erst danach zusätzlich ein Teil des Fluidraums zur Verdrängung des ersten Fluids ausgefüllt wird.

Dies kann beispielsweise dadurch bewerkstelligt werden, dass das erste Sperrelement derart eingerichtet ist, dass beim Übergang vom ersten eingestellten Volumen des Sperrelements zum zweiten eingestellten Volumen zunächst der Einströmkanal/Ausströmkanal gesperrt wird und danach bei weiterer Volumenzunahme und dem Übergang zum dritten eingestellten Volumen ein Teil des Volumens im Fluidraum verdrängt wird.

Beispielsweise kann vorgesehen sein, dass das Sperrelement verschiedene Wandungsbereiche aufweist, die durch Unterschiede des Materials und/oder der Materialstärke unterschiedlich expandierbar sind. In diesem Fall ist beispielsweise der Teil des Sperrelements, der den Einströmkanal verschließen soll, zur leichteren Expansion mit einer geringeren Wandstärke oder einem schwächeren Material realisiert als der Teil des Sperrelements, der sich in den Fluidraum hinein ausdehnen soll. Das entsprechende Sperrelement ebenso wie gegebenenfalls zusätzliche Verdrängungselemente sind aus einem elastisch dehnbaren Material, bei Kontakt mit Lebewesen aus einem biokompatiblen Material wie zB. Polyurethan hergestellt.

Es kann auch zusätzlich oder alternativ dazu vorgesehen sein, dass das Sperrelement zumindest in einem Zustand, in dem es den Einström-/Ausströmkanal weitestgehend sperrt und noch ein minimales Volumen des Fluidraumes verdrängt, insbesondere auch im entspannten Zustand, eine sich zum Fluidraum hin querschnittsmäßig verjüngende Form aufweist, wobei die breite Basis des Sperrelementes an dem versperrbaren Einström-/Ausströmkanal angeordnet ist. Diese Form kann dann gegebenenfalls noch weiter expandiert werden, bis dass das Element den Fluidraum weitgehend ausfüllt, und bei Vorhandensein eines Gehäuses möglichst weitgehend an der Innenwand des Gehäuses anliegt.

Ist kein Gehäuse vorgesehen, so kann der Fluidraum auch beispielsweise durch die Wände eines Blutgefäßes begrenzt sein.

Ein gegebenenfalls vorgesehenes Gehäuse kann im Übrigen vorteilhaft auch komprimierbar und expandierbar sein, um im komprimierten Zustand in den Körper eines Patienten eingebracht und dort im expandierten Zustand betrieben zu werden. Wichtig ist dabei, dass das Gehäuse so weit stabilisiert wird, dass es auch in der Ansaugphase der Fluidpumpe, wenn entsprechende Verdrängungskörper im Fluidraum kollabieren, durch den entstehenden Unterdruck nicht ebenfalls kollabiert.

Erfindungsgemäß ist, unabhängig von der Ausführungsform der Fluidpumpe, also beispielsweise auch bei einer Pumpe mit einem Rotor, vorgesehen, dass das Gehäuse bistabil mit einem expandierten Zustand und einem komprimierten Zustand ausgebildet ist.

Dies kann derart realisiert sein, dass das Gehäuse einen gewölbten Bereich aufweist, der zwischen einem nach außen gewölbten Zustand und einem nach innen gewölbten Zustand veränderbar ist.

Zudem kann vorgesehen sein dass der gewölbte Bereich teilzylindrisch ist. Er kann sich über die gesamte Länge des Gehäuses erstrecken. Es ist auch eine kalottenartige bistabile Wölbung in diesem Zusammenhang denkbar.

Die entsprechende Versteifung eines Gehäuses kann auch durch knickbare Verstärkungsstege erreicht werden, die durch geeignete mechanische Einwirkung zum Kollabieren des Gehäuses einknickbar sind. Das Gehäuse soll mindestens im Gebrauchszustand einen formstabilen Zustand besitzen. Denkbar sind wie beschrieben, zwei formstabile Zustände jeweils im Gebrauchzustand (exapandiert) und bei einer minimalinvasiven Implantation (komprimiert). Mögliche Ausführungen sind vorrangig als unelastische oder wenig elastische schlauchförmige Bauteile, Materialien z.B. PET, mit ggf. einer Stützstruktur aus dem gleichen oder anderem Material, z.B auch superelastischem Metall wie Nitinol, hergestellt.

Eine weitere vorteilhafte Ausgestaltung der Erfindung sieht vor, dass der Fluidraum einen Verbindungskanal zwischen dem Einströmkanal und dem Ausströmkanal bildet und dass die Fördereinrichtung im Verbindungskanal ein bezüglich seines Volumens steuerbares Verdrängungselement aufweist, das den Verbindungskanal wenigstens abschnittsweise bei einem ersten eingestellten Volumen freigibt und bei einem zweiten eingestellten Volumen wenigstens überwiegend ausfüllt.

Diese Ausführungsform weist einzeln ansteuerbare Elemente der Fördereinrichtung auf, die sukzessive bezüglich ihres Volumens veränderbar sind und damit bestimmte Volumina einnehmen, so dass in der gewünschten Reihenfolge zunächst ein Zugangskanal (Einström- und Ausströmkanal) des Fluidraums gesperrt und danach in gewünschtem zu- oder abnehmendem Maß das Volumen des Fluidraums, das dem ersten Fluid zugänglich ist, sukzessive verkleinert oder vergrößert wird. Dies geschieht vorteilhaft entlang des Verbindungskanals, der den Fluidraum bildet, so dass in quasi peristaltischer Art das im Fluidraum befindliche erste Fluid verdrängt bzw. verschoben werden kann. Durch die einzelne Ansteuerbarkeit der Sperrelemente bzw. eines Verdrängungselements oder mehrerer Verdrängungselemente ist eine besonders dedizierte und auf das gewünschte Durchfluss- bzw. Druckprofil abgestellte Abfolge der Volumenänderungen realisierbar.

Zum Ansaugen von Fluid wird dazu ein Sperrelement im Ausströmkanal derart aktiviert, dass es den Ausströmkanal sperrt, so dass von dort kein Fluid angesaugt werden kann und dies bei geöffnetem Einströmkanal von dessen Seite her angesaugt wird. In der Ansaugphase werden die in dem Verbindungskanal befindlichen Verdrängungselemente maximal kollabiert, so dass möglichst viel Fluid in den Fluidraum einströmen kann.

Danach wird mittels des im Einströmkanal befindlichen Sperrelements dieser versperrt, das im Ausströmkanal befindliche Sperrelement kollabiert, so dass der Ausströmkanal geöffnet ist, und danach werden die Verdrängungselemente expandiert, und zwar beginnend mit demjenigen Verdrängungselement, das dem Einströmkanal am nächsten ist.

Sind mehrere Verdrängungselemente vorgesehen, so kann jedes einzelne Verdrängungselement den Verbindungskanal abschnittsweise ganz ausfüllen und abdichten. Wird dann das folgende Verdrängungselement ebenfalls expandiert, und ist der Verbindungskanal vollständig ausgefüllt, so kann danach das erste Verdrängungselement wieder kollabieren oder auch expandiert bleiben. Im ersten Fall ergibt sich ein wandernder Bereich maximaler Expansion entlang des Verbindungskanals. Bei der zweiten Variante wird der Verbindungskanal sukzessive vollständig durch die Verdrängungselemente ausgefüllt.

Hierzu können wenigstens zwei, jedoch auch drei, vier oder fünf oder noch mehr Verdrängungselemente entlang des Verbindungskanals vorgesehen sein.

Diese können jeweils ballonartig elastisch expandierbar sein, beispielsweise in Form von kugel- oder zylinderförmigen Elastomerballons.

Zur individuellen Ansteuerung der einzelnen Sperrelemente und Verdrängungselemente können diese zur Erzeugung einer quasi- peristaltischen Verdrängungsbewegung jeweils einzeln über Druckleitungen steuerbar mit einer gemeinsamen Druckquelle oder mit mehreren Druckquellen verbindbar sein. Damit kann über eine entsprechend gestaltete Ventileinrichtung jede gewünschte Abfolge von Volumenzunahmen und -abnahmen in den einzelnen Elementen der Fördereinrichtung erreicht werden. Als Fluid zur Füllung der Elemente der Fördereinrichtung kann entweder ein Gas oder eine Flüssigkeit, insbesondere eine körperverträgliche Flüssigkeit wie eine Salzlösung verwendet werden.

Vorteilhaft kann vorgesehen sein, dass die Druckleitungen ausreichend steif sind und sdie Fluidpumpe oder einzeln deren Elemente in bezug auf ihre Position, insbesondere in und entgegen der Strömungsrichtung fixieren.

Die Elemente der Fördereinrichtung können auch untereinander über eine Druckleitung verbunden sein, die beispielsweise Ventile oder Drosselstellen aufweisen kann, um bei der Ansteuerung ein bestimmtes Zeitprofil des Druckverlaufs in einzelnen Elementen zu erreichen.

Die Offenbarung bezieht sich zudem auf ein Verfahren zum Betrieb einer Fluidpumpe. Dabei ist nach der Bauart und Ausführungsform der Fluidpumpe zu unterscheiden, welches Betriebsverfahren besonders geeignet ist.

Es kann dazu vorgesehen sein, dass sowohl das in dem Fluidraum durch das erste Fluid ausfüllbare Volumen durch steuerbare Volumenänderung eines Elements einer Fördereinrichtung veränderbar ist, als auch Sperrelemente im Ein- und Ausströmkanal durch Volumenänderung zwischen der Sperrstellung und einer Durchlassstellung verändert werden können, wobei durch zykische Volumenänderung der Sperr- und Verdrängungskörper eine zyklisch fortschreitende Querschnittsverengung und -erweiterung des für das Fluid im Fluidraum zur Verfügung stehenden Volumens erfolgt.

Ist ein Sperrelement im Einströmkanal vorgesehen, das sich zusätzlich in den Fluidraum hinein ausdehnen kann, so kann auch als vorteilhaftes Betriebsverfahren vorgesehen sein, dass ein erstes und ein zweites Sperrelement derart angesteuert werden, dass zunächst das im Ausströmkanal angeordnete Sperrelement diesen freigibt, danach das im Einströmkanal angeordnete Sperrelement diesen versperrt und danach das im Einströmkanal angeordnete Sperrelement durch weitere Expansion wenigstens einen Teil des Volumens des Fluidraums verdrängt.

Zudem kann dabei vorteilhaft vorgesehen sein, dass danach das im Ausströmkanal angeordnete Sperrelement diesen versperrt und das im Einströmkanal angeordnete Sperrelement diesen sowie das im Fluidraum zusätzlich verdrängte Volumen freigibt.

Diese beschriebene Abfolge kann dann zyklisch wiederholt werden, um einen pulsierenden Pumpvorgang mit der gewünschten Frequenz und dem gewünschten Pumpvolumen zu erzielen.

Ist eine Fluidpumpe mit wenigstens einem Sperrelement und gesondert von diesem ansteuerbaren Verdrängungselementen im Fluidraum vorgesehen, so sieht ein besonders vorteilhaftes Betriebsverfahren für eine solche Pumpe vor, dass die Sperrelemente und das/die Verdrängungselement(e) derart angesteuert wird/ werden, dass bei einem fluidgefüllten Fluidraum das Sperrelement am Einströmkanal und die Verdrängungselemente bei geöffnetem Ausströmkanal sukzessive derart bezüglich ihres Volumens expandiert werden, dass das für das Fluid freie Volumen im Fluidraum von der Einströmseite her verkleinert wird, dass danach das Sperrelement im Ausströmkanal expandiert wird, so dass es den Ausströmkanal im Wesentlichen verschließt, und dass darauf das/die Verdrängungselement(e) und das Sperrelement im Einströmkanal komprimiert werden. Dabei kann zudem vorgesehen sein, dass das Sperrelement zeitlich vor den Verdrängungselementen komprimiert wird. Der gesamte Vorgang kann zyklisch wiederholt werden.

Die genannte zeitlich gestaffelte Ansteuerung der Sperr- und Verdrängungselemente erzeugt eine Art peristaltischer Förderung des Fluids über den Einströmkanal in den Fluidraum hinein, durch diesen hindurch und aus dem Ausströmkanal hinaus.

Im Folgenden wird die Erfindung anhand eines Ausführungsbeispiels in einer Zeichnung gezeigt und anschließend beschrieben.

Dabei zeigt
- Fig. 1: grundsätzlich die Funktion und Anwendung einer in einem Blutgefäß betreibbaren Mikropumpe,
- Fig. 2: in einem Längsschnitt und einem Querschnitt eine Fluidpumpe in einer ersten Ausführungsform mit zwei Sperrelementen und einem Verdrängungselement,
- Fign. 3―6: die Pumpe aus Fig. 2 in verschiedenen Pumpzuständen,
- Fign. 7,7a,7b-9: eine weitere Ausführungsform einer Fluidpumpe mit einem Sperrelement im Einströmkanal, das auch als Verdrängungselement wirkt,
- Fig. 10: ein Ablaufdiagramm einer weiteren Ausführungsform der Pumpe mit einer Mehrzahl von Verdrängungselementen,
- Fig. 11: eine Ausführungsform einer Fluidpumpe mit mehreren Verdrängungselementen in einem schematischen Ablaufplan der Ansteuerung in Variation zur Fig. 10,
- Fig. 12: einen schematischen Anschlussplan von Sperr- und Verdrängungselementen mit einer Druckquelle und
- Fig.13-15: verschiedenen Zustände eines Gehäuses einer Fluidpumpe.

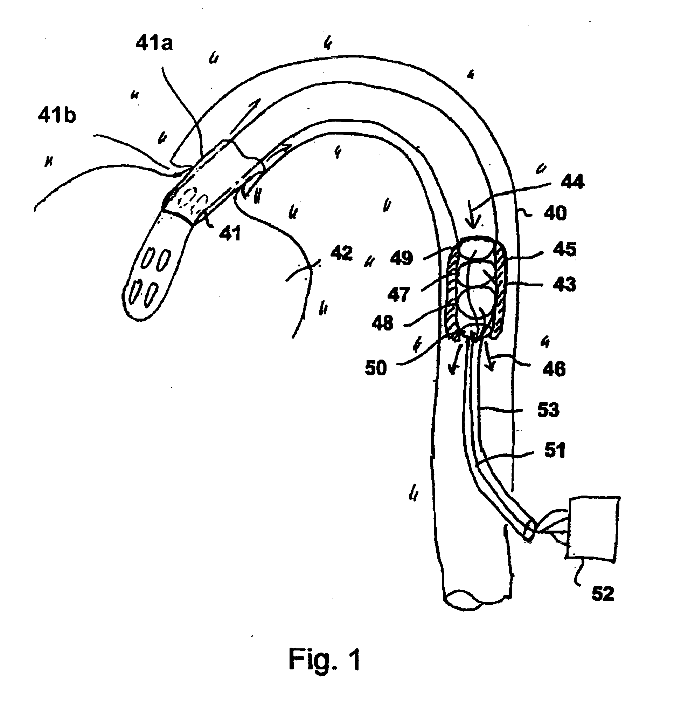

Fig. 1 zeigt schematisch in einem Überblick die Anwendung einer erfindungsgemäßen Fluidpumpe beim Einsatz in einem Blutgefäß eines menschlichen Körpers in Herznähe. Das Blutgefäß ist in der Figur mit 40 bezeichnet. In dem Blutgefäß kann optional ein Ansaugschlauch 41 von einer Herzkammer 42 in Richtung des Pfeils 44 verlaufen, der in ein Pumpengehäuse 43 münden kann, sofern ein Pumpengehäuse gesondert vorgesehen und der Fluidraum nicht durch die Gefäßwände des Blutgefäßes begrenzt ist. Der Ansaugschlauch kann auch mit einem diesen im Bereich der Herzklappen umgebenden schlaffen, beispielsweise aus einer Folie bestehenden Abströmschlauch 41a umgeben sein, so dass zwischen diesem und dem Schlauch im Ansaugfall, wenn die Herzklappenventile 41b geöffnet sind, leicht Blut passieren kann. In der umgekehrten Flussrichtung, in die Herzkammer hinein, schließen jedoch die Herzklappen und drücken gleichzeitig den Abströmschlauch an den Ansaugschlauch an, so dass der Zwischenraum verschlossen und das Blut am Rückfluss gehindert wird.

Die Fluidpumpe weist beispielsweise ein im Wesentlichen rotationssymmetrisches oder ovales oder elliptisches Gehäuse 45 auf, das in seinem Inneren einen Fluidraum in Form eines Verbindungskanals bildet, und zwar zwischen dem Einströmkanal, der der Herzkammer 42 zugewandt ist, und dem Ausströmkanal, durch den das Blut in Richtung der Pfeile 46 ausströmt.

In dem Fluidraum der Fluidpumpe sind Verdrängungskörper 47, 48 sowie ein Sperrkörper 49 im Einströmkanal und ein Sperrkörper 50 im Ausströmkanal vorgesehen. Diese sind mittels Fluiddruckleitungen 51 mit einer Druckquelle 52 steuerbar verbunden, so dass die Sperrkörper und die Verdrängungskörper wunschgemäß expandiert und kollabiert werden können.

Durch geeignete Ansteuerung der Elemente der Fördereinrichtung wird Blut aus der Herzkammer 42 abgesaugt und stromabwärts der Fluidpumpe 43 in das Blutgefäß 40 abgegeben.

Die Fluidpumpe ist am Ende eines Hohlkatheters 53 angeordnet, durch dessen Lumen die Druckleitungen 51 zum Körperäußeren hin verlaufen und der durch eine Schleuse aus dem Blutgefäß 40 zum Körperäußeren hinausgeführt ist.

Im Folgenden wird auf den Aufbau und die Betriebsweise der Fluidpumpe in verschiedenen Bauvarianten genauer eingegangen.

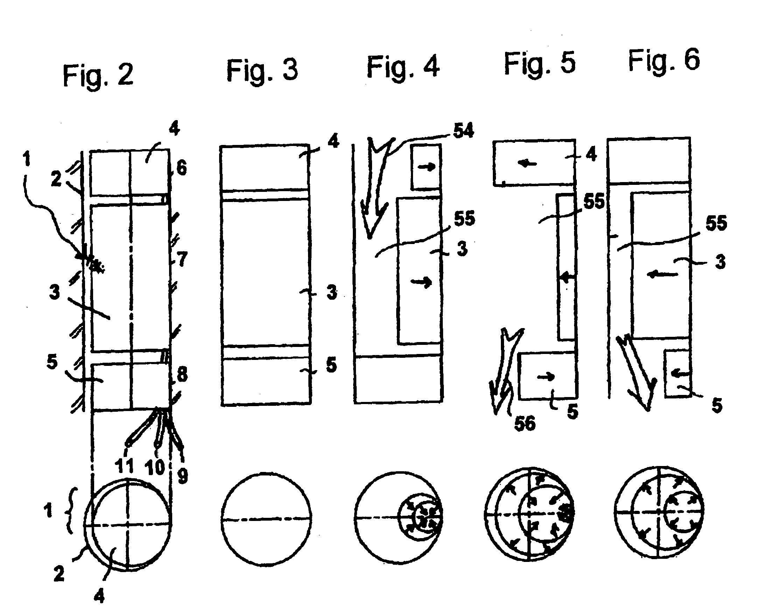

In der Fig. 2 ist die Innenkontur eines Gehäuses einer Fluidpumpe oder beispielsweise, falls kein Gehäuse vorgesehen ist, eines Blutgefäßes mit 2 bezeichnet. Diese begrenzt den innen liegenden Fluidraum, der in der Darstellung im Wesentlichen von dem Verdrängungselement 3 ausgefüllt wird. Das Gehäuse ist im Wesentlichen durch einen undehnbaren Schlauch, beispielsweise aus einem Kunststoff, gebildet.

Der Einströmkanal ist durch das Sperrelement 4 im Wesentlichen ausgefüllt, während der Ausströmkanal durch das Sperrelement 5 ausgefüllt ist.

Die Verdrängungselemente und Sperrelemente sind im Wesentlichen zylindrisch ausgebildet und bestehen beispielsweise aus einem Elastomermaterial. Sie sind an zumindest einer Seite über Verbindungsstellen 6, 7, 8 mit der Innenwand des Gehäuses 2 verbunden und darüber positioniert.

Die Verdrängungskörper 3 sowie die Sperrkörper 4, 5 sind jeweils über Druckleitungen 9, 10, 11 mit einer nicht näher dargestellten Druckquelle verbunden.

Mit 1 ist die Fördereinrichtung der Fluidpumpe im Ganzen bezeichnet.

Im unteren Teil der Figur ist eine Ansicht in Axialrichtung vom Ende des Sperrelements 5 aus gesehen dargestellt.

Die Darstellungen der Fign. 3, 4, 5 und 6 betreffen dieselbe Fluidpumpe wie die Fig. 2 in verschiedenen Stadien des Pumpvorgangs. In der Fig. 3 ist dargestellt, dass sowohl die Sperrelemente 4, 5 als auch das Verdrängungselement 3 den Innenraum des Gehäuses vollständig ausfüllen. Damit ist das gesamte Fluid aus dem Fluidraum verdrängt.

Gemäß Fig. 4 wird das Verdrängungselement 3 kollabiert, ebenso wie das Sperrelement 4, so dass das Fluid in Richtung des Pfeils 54 in den Fluidraum 55 einströmen kann.

Gemäß Fig. 5 ist das Verdrängungselement noch weiter kollabiert, so dass fast der gesamte Innenraum des Gehäuses als Fluidraum 55 für das Fluid zur Verfügung steht und gefüllt ist. Gleichzeitig ist das Sperrelement 4 so weit expandiert, dass es den Einströmkanal fast vollständig ausfüllt und sperrt. Zudem wird das Sperrelement 5 im Ausströmkanal kollabiert, so dass dort eine Möglichkeit für das Fluid entsteht, in Richtung des Pfeils 56 aus dem Gehäuse der Fluidpumpe auszuströmen.

In der Fig. 6 ist dargestellt, dass das Sperrelement 5 weitestgehend kollabiert ist und gleichzeitig das Verdrängungselement 3 weiter expandiert wird, um den Fluidraum 55 zu verkleinern und aus diesem das Fluid zum Ausströmkanal hin zu verdrängen. Danach wird das Sperrelement 5 wieder expandiert und das Verdrängungselement 3 gemeinsam mit dem Sperrelement 4 kollabiert.

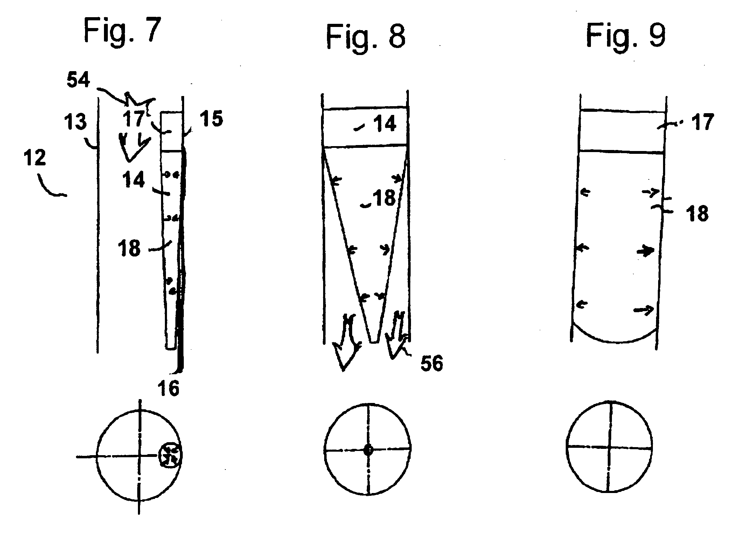

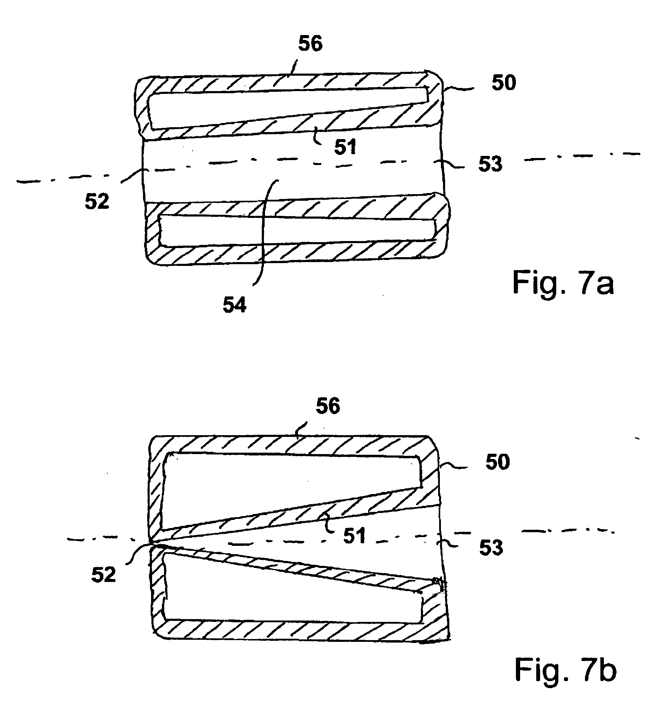

Die Fign. 7, 8 und 9 beziehen sich auf eine andere Ausführungsform der Fluidpumpe, bei der die Innenkontur des Gehäuses mit 13 bezeichnet ist und die Fördereinrichtung insgesamt mit 12.

Es ist ein einziger volumenveränderbarer Körper vorgesehen, der einen zylindrischen Sperrbereich 17 und einen Verdrängungsbereich 18 aufweist und im Ganzen im drucklosen Zustand zylindrisch, ausgebildet ist. Der Sperrbereich 17 dehnt sich während des Druckaufbaus zylindrisch aus , während aufgrund der zum verjüngten Bereich verdickten Wandstärke der Verdrängungsbereich 18 zwischenzeitlich einen Konus ausbildet, bis auch dieser Bereich maximal aufgepumpt ist und den Querschnitt des Fluidraums voll ausfüllt. Der kombinierte Sperr-/ Verdrängungskörper ist im Bereich 15 mit der Innenkontur des Gehäuses verbunden.

Die Figur 7a zeigt einen aufpumpbaren hohlzylindrischen aufpumpbaren Sperr-/Verdrängungskörper (50), der an der inneren Mantelfläche (51) ein elastisches Material mit einer Wandstärke aufweist, die sich von einer geringen Dicke an dem dem Einströmkanal(52) zugewandten Ende auf dem Weg zu seinem dem Abströmkanal (53) zugewandten Ende zu einer größeren Dicke hin ändert.

Damit ergibt sich im entspannten Zustand ein zylindrischer Innenraum (54), der sich bei ansteigendem Innendruck im aufpumpbaren Hohlraum des Körpers zuerst am einströmseitigen Ende verengt, bis dass der Durchgang dort vollständig verschlossen ist. Erst nachfolgend dehnen sich die übrigen Teile der Innenwand nach innen aus und füllen einen größeren Teil des gesamten Innenraums aus, wie in Figur 7b dargestellt. Die Äu-βere Wand (56) des Hohlzylinders vorteilhaft aus einem weniger oder nicht elastischen Stoff oder weist eine durchgehend größere Wanddicke auf als die innere Wand.

In der Fig. 7 ist der volumenveränderliche Körper im Wesentlichen kollabiert, so dass im Bereich des Fluidraumes, wie mittels des Pfeils 54 dargestellt, Fluid einströmen kann. Vorteilhaft ist in dieser Konfiguration ein Ventil oder ein weiterer Sperrkörper am ausströmseitigen Ende, um das Einströmen von Fluid aus dieser Richtung zu verhindern.

Es ist zudem eine Druckleitung 16 vorgesehen, die mit dem Sperr-/Verdrängungskörper 14, 17, 18 verbunden ist. Wird über diesen ein Fluid zugeführt, so dehnt sich zuerst der Sperrbereich 17 aus, wie in der Fig. 8 dargestellt. Der Einströmkanal wird bei genügender Ausdehnung des Sperrbereichs vollständig verschlossen, so dass an diesem Ende das Fluid aus dem Fluidraum nicht ausströmen kann. Nachfolgend wird der Verdrängungsbereich 18 konusförmig weiter expandiert, so dass das Fluid in Richtung der Pfeile 56 ausströmt. Dies geschieht so lange, bis, wie in Fig. 9 dargestellt, der Fluidraum gänzlich durch den kombinierten Sperr-/ Verdrängungskörper ausgefüllt worden ist. Im unteren Bereich der Fluidpumpe sollte für einen kontinuierlichen Betrieb noch ein Ausströmventil bzw. ein Sperrkörper vorgesehen sein, der verhindert, dass in der Ansaugphase durch den Ausströmkanal Fluid angesaugt wird.

Im unteren Bereich der Fign. 7, 8, 9 ist jeweils eine Draufsicht auf die Fluidpumpe vom Ausströmkanal her dargestellt.

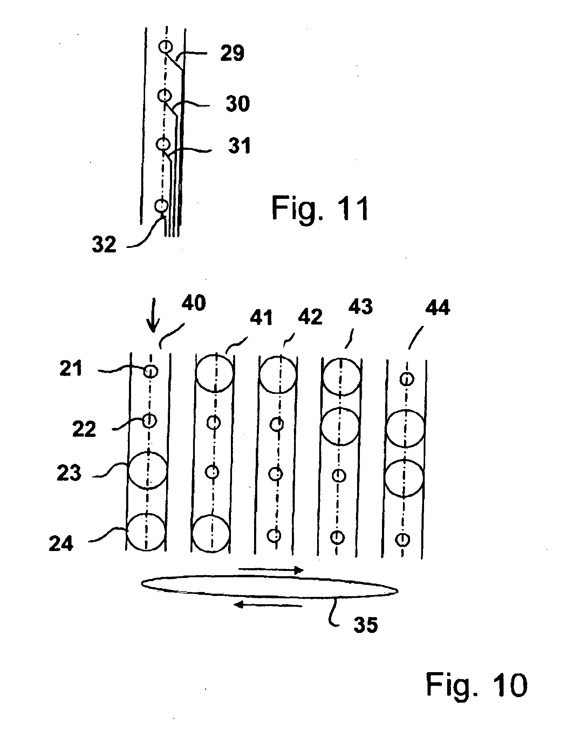

Fig. 10 zeigt schematisch in Längsschnitten den Aufbau einer Fluidpumpe mit einem langgestreckten Fluidraum 20, der einen zylindrischen Verbindungskanal zwischen dem Einströmkanal, dargestellt durch den Pfeil 19, und dem Ausströmkanal, beispielhaft dargestellt durch den Pfeil 33, bildet, wobei das Gehäuse als undehnbarer oder im Wesentlichen undehnbarer Schlauch ausgebildet ist.

Die Figur zeigt nebeneinander von links nach rechts gestaffelt fünf verschiedene Zustände der Sperrkörper und Verdrängungskörper im Inneren des Gehäuses. Zunächst sind als Sperrkörper im Einströmkanal der Ballon 21 und im Ausströmkanal der Ballon 24 vorgesehen. Zwischen diesen sind als reine Verdrängungselemente die Ballons 22 und 23 angeordnet.

Alle Ballons können im Wesentlichen baugleich ausgeführt sein. Sie sind, wie in Figur 11 ersichtlich, mit Druckleitungen 29, 30, 31, 32 versehen und mittels dieser jeweils steuerbar mit einer Druckquelle verbunden, die nicht näher dargestellt ist, die jedoch gezielt steuerbar das Aufpumpen/Dilatieren und Kollabieren jedes einzelnen Ballons zu einer gewünschten Zeit erlaubt.

Es können Verbindungen vorgesehen sein, über die die Ballons mit der Schlauchwand verbunden sind, um sie in axialer Richtung zu fixieren. Es ist jedoch auch denkbar, die Ballons an einem Strang oder einer Schiene zu befestigen und gemeinsam in das Gehäuse einzuziehen und mittels des Strangs an diesem zu positionieren. Dabei kann der Strang auch dazu dienen, die Druckleitungen zu fixieren.

Die einzelnen Stadien beim Betrieb der Pumpe sind in der Fig. 10 von 40 bis 44 durchnummeriert. Im Zustand 40 sind die Ballons 21 und 22 kollabiert, so dass der Einströmkanal frei ist und der einströmseitige Bereich des Fluidraums mit dem zu fördernden Fluid gefüllt ist. Im Status 41 ist das Sperrelement 21 im Einströmkanal voll expandiert, so dass der Einströmkanal gesperrt ist. Der Ausströmkanal ist in dieser Konstellation noch durch den Ballon 24 gesperrt.

Im Zustand 42 ist der Ballon 24 kollabiert, so dass das Fluid durch den Ausströmkanal abströmen kann. Eine weitere Verringerung des Volumens findet beim Übergang zum Zustand 43 statt, wenn der zweite Verdrängungskörper 22 ebenfalls expandiert ist. Beim Übergang zum Zustand 44 wird der Ballon 23 expandiert, so dass das Fluid weitergetrieben wird. Gleichzeitig ist der einströmseitige Ballon 21 wieder kollabiert, so dass dort neues Fluid einströmen kann. Der nächste Zustand ist wieder der Zustand 40, in dem alles Fluid aus dem Fluidraum ausgepresst ist. Nun kann der Zyklus, wie im Status 40 dargestellt, von neuem beginnen. Damit ist ein pulsatiler Betrieb der Pumpe durch einen quasi peristaltischen Effekt bei gleichbleibendem Außendurchmesser des Pumpenaufbaus möglich.

In der Fig. 11 sind zusätzlich zur Darstellung der Figur 10 lediglich die Druckleitungen 29,30,31,32 zu den Ballons 21,22,23,24 dargestellt.

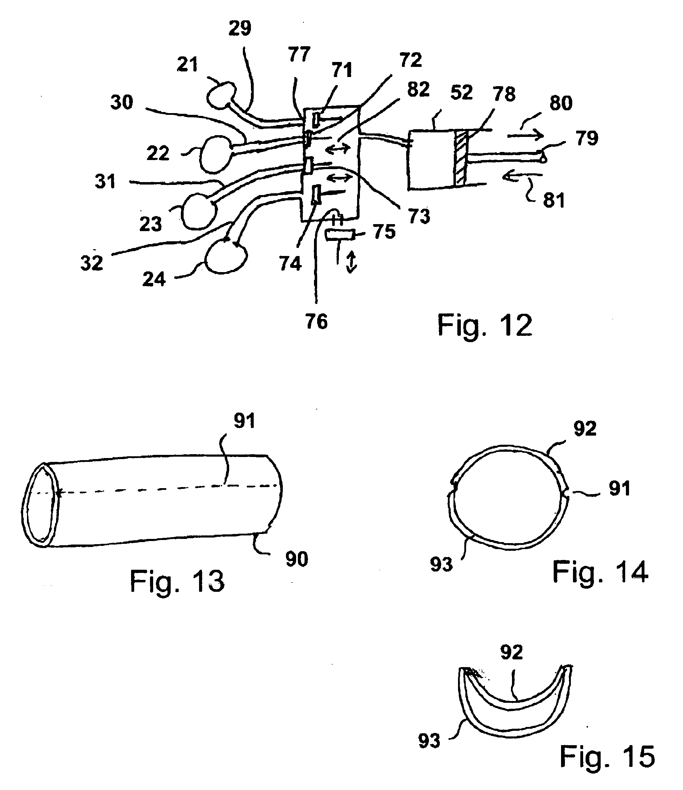

Figur 12 zeigt einen schematischen Anschlussplan einer Druckquelle (53) an Druckleitungen (29,30,31,32) von Sperr- und Verdrängungskörpern(21,22,23,24). Die Druckquelle weist einen in einem Zylinder bewegbaren Kolben (78) auf, der je nach Bewegungsrichtung in Richtung des Pfeils 81 Überdruck, in Richtung des Pfeils 80 Unterdruck erzeugt. Der Druck kann mittels steuerbarer Ventile (71,72,73,74 über Kanäle (77) zwischen dem Verbindungsraum (82) und den Drückleitungen ausgeglichen werden. Dadurch kann steuerbar in allen Sperr- und Verdrängungskörpern (21,22,23,24) Über- oder Unterdruck erzeugt werden.

Figur 13 zeigt ein zylindrisches Pumpengehäuse (90), das einen geschwächten Wandbereich 91 (gestrichelt dargestellt) aufweist, in dem der nach außen gewölbte Bereich 92 umklappen kann. Wie aus den Figuren 14 und 15 ersichtlich, kann der Bereich 92 nach innen geklappt werden, um die Außenmaße des Gehäuses zu verringern (Transportzustand). Später kann der Bereich 92 wieder aus dem Basisbereich 93 des Gehäuses nach außen geklappt werden, um das Gehäuse zu expandieren. Das Gehäuse ist in beiden Zuständen eigenstabil und kann beispielsweise durch Druckstöße, jedoch alternativ auch durch Temperaturänderungen, umgeklappt werden, wenn beispielsweise der Bereich 92 aus einem Bimetall oder einer Gedächtnislegierung besteht.

Durch die Erfindung wird in den verschiedenen Ausgestaltungen ein pulsierender Pumpenbetrieb mit hoher Zuverlässigkeit und großem Durchsatz ermöglicht, ohne dass rotierende Teile erforderlich sind, wobei die gesamte Steuerung der Pumpe über Druckbeaufschlagung mit einem Fluid erfolgen kann. Dies kann den Pumpenbetrieb innerhalb eines Körpers eines Lebewesens erleichtern. Um die Versorgung der einzelnen Sperr- und Verdrängungskörper mit Druckleitungen zu vereinfachen, können diese im Bereich der Pumpe zu einem gemeinsamen Strangkörper zusammengeführt werden, der beispielsweise einstückig ausgeführt und mit der erforderlichen Anzahl von Lumina versehen sein kann, so dass die Führung innerhalb des Hohlraums eines Katheters besonders einfach wird. Am proximalen Ende des Katheters kann dieser strangförmige Körper dann wieder in verschiedene Druckleitungen aufgeteilt werden, die einer Ventilsteuerungseinheit zugeführt werden können.

## Patentansprüche

1. Fluidpumpe für ein erstes Fluid mit einem Fluidraum, mit wenigstens einem Einströmkanal und wenigstens einem Ausströmkanal für das erste Fluid, wobei
a) das in dem Fluidraum durch das erste Fluid ausfüllbare Volumen durch steuerbare Volumenänderung eines Elements einer Fördereinrichtung veränderbar ist, wobei die Fördereinrichtung wenigstens ein bezüglich seines Volumens steuerbares Sperrelement (4,5,14,17,18,21,24) aufweist, das einen Einström- und/oder einen Ausströmkanal des Fluidraums bei einem ersten eingestellten Volumen des Sperrelements wenigstens überwiegend, insbesondere ganz, freigibt und den entsprechenden Kanal bei einem zweiten eingestellten Volumen des Sperrelements (4, 5, 14, 17, 18, 21, 24) wenigstens überwiegend, insbesondere ganz, sperrt, oder
b) die Fluidpumpe eine Pumpe mit einem Rotor ist,
**dadurch gekennzeichnet, dass** der Fluidraum von einem Gehäuse (43,90) umgeben ist, das bistabil ausgebildet ist und einen stabilisierten expandierten Betriebszustand und wenigstens einen weiteren, komprimierten Zustand aufweist.

2. Fluidpumpe nach Anspruch 1, **dadurch gekennzeichnet, dass** das Gehäuse (43,90) einen gewölbten Bereich (92) aufweist, der zwischen einem nach außen gewölbten Zustand und einem nach innen gewölbten Zustand veränderbar ist.

3. Fluidpumpe nach Anspruch 2, **dadurch gekennzeichnet, dass** der gewölbte Bereich (92) teilzylindrisch ist.

4. Fluidpumpe nach Anspruch 1 oder einem der folgenden, **dadurch gekennzeichnet, dass** das/die volumensteuerbare(n) Element(e) (4,5,14,17,18,21,22,23,24) jeweils einen mit einem zweiten Fluid füllbaren Hohlraum aufweist/ aufweisen.

5. Fluidpumpe nach Anspruch 1 oder einem der folgenden, **dadurch gekennzeichnet, dass** das/die volumensteuerbare(n) Element(e) (4,5,14,17,18,21,22,23,24) jeweils eine wenigstens teilweise elastische Außenwand aufweist/aufweisen.

6. Fluidpumpe nach Anspruch1 oder einem der folgenden, **dadurch gekennzeichnet, dass** wenigstens ein Sperrelement (4,5,14,17,18,21,24) bezüglich seines Volumens auf ein drittes Volumen ausgedehnt werden kann, bei dem es über den beim ersten und zweiten Volumen eingenommenen Raum hinaus wenigstens einen Teil des Fluidraums ausfüllt.

7. Fluidpumpe nach Anspruch 1 oder einem der folgenden, **dadurch gekennzeichnet, dass** das erste Sperrelement (14,17,18) derart eingerichtet ist, dass beim Übergang vom ersten eingestellten Volumen des Sperrelements zum zweiten eingestellten Volumen zunächst der Einströmkanal/Ausströmkanal gesperrt wird und danach bei weiterer Volumenzunahme und dem Übergang zum dritten eingestellten Volumen ein Teil des Volumens im Fluidraum verdrängt wird.

8. Fluidpumpe nach Anspruch 6 oder 7, **dadurch gekennzeichnet, dass** das Sperrelement (4,5,14,17,18,21,24) verschiedene Wandungsbereiche aufweist, die durch Unterschiede des Materials und/oder der Materialstärke unterschiedlich expandierbar sind.

9. Fluidpumpe nach Anspruch 7 oder 8, **dadurch gekennzeichnet, dass** das Sperrelement (4,5,14,17,18,21,24) zumindest in einem Zustand, in dem es den Einströmkanal weitestgehend sperrt und noch ein minimales Volumen des Fluidraumes verdrängt, insbesondere auch im entspannten Zustand, eine sich zum Fluidraum hin querschnittsmäßig verjüngende Form aufweist, wobei die breite Basis des Sperrelementes an dem versperrbaren Einström-/Ausströmkanal angeordnet ist.

10. Fluidpumpe nach Anspruch 1 oder einem der folgenden , **dadurch gekennzeichnet, dass** der Fluidraum einen Verbindungskanal zwischen dem Einströmkanal und dem Ausströmkanal bildet und dass die Fördereinrichtung im Verbindungskanal ein bezüglich seines Volumens steuerbares Verdrängungselement (3,22,23) aufweist, das den Verbindungskanal wenigstens abschnittsweise bei einem ersten eingestellten Volumen freigibt und bei einem zweiten eingestellten Volumen wenigstens überwiegend ausfüllt.

11. Fluidpumpe nach Anspruch 10, **dadurch gekennzeichnet, dass** die Fördereinrichtung zusätzlich zu dem Verdrängungselement (3,22,23) sowohl im Einströmkanal als auch im Ausströmkanal jeweils ein Sperrelement (4, 5, 21, 24) aufweist.

12. Fluidpumpe nach Anspruch 10 oder 11, **dadurch gekennzeichnet, dass** die Fördereinrichtung wenigstens zwei, insbesondere drei, vier oder fünf oder mehr als fünf einzeln steuerbare Verdrängungselemente (3,22,23) aufweist.

13. Fluidpumpe nach Anspruch 10, 11 oder 12, **dadurch gekennzeichnet, dass** das/die Verdrängungselement(e) (3,22,23) ballonartig elastisch expandierbar ist/sind.

14. Fluidpumpe nach Anspruch 13, **dadurch gekennzeichnet, dass** das/die Verdrängungselement(e) (3,22,23) zylindrisch oder kugelförmig ist/sind.

15. Fluidpumpe nach Anspruch 10 oder einem der folgenden, **dadurch gekennzeichnet, dass** die Sperrelemente und die Verdrängungselemente (3,22,23) über einzelne Druckleitungen (26,27) steuerbar mit einer Druckquelle (52) verbunden sind.

## Claims

1. A fluid pump for a first fluid with a fluid space, with at least one inflow channel and at least one outflow channel for the first fluid, wherein
a) the volume that can be filled in the fluid space by the first fluid can be changed by controllable volume change of an element of a conveying arrangement, wherein the conveying arrangement comprises at least one blocking element (4, 5, 14, 17, 18, 21, 24) of which the volume can be controlled and which releases at least predominantly, in particular completely, an inflow channel and/or outflow channel of the fluid space with a first set volume of the blocking element and at least predominantly, in particular completely, blocks the corresponding channel with a second set volume of the blocking element (4, 5, 14, 17, 18, 21, 24), or
b) the fluid pump is a pump with a rotor,
**characterised in that** the fluid space is surrounded by a housing (43, 90) which is bistable and has a stabilised expanded operating state and at least one further state which is a compressed state.

2. The fluid pump according to claim 1, **characterised in that** the housing (43, 90) comprises a curved region (92) which can be changed between an outwardly curved state and an inwardly curved state.

3. The fluid pump according to claim 2, **characterised in that** the curved region (92) is partly cylindrical.

4. The fluid pump according to claim 1 or one of the claims thereafter, **characterised in that** the volume-controllable element (4, 5, 14, 17, 18, 21, 22, 23, 24) or each of the volume-controllable elements has a hollow space that can be filled with a second fluid.

5. The fluid pump according to claim 1 or one of the claims thereafter, **characterised in that** the volume-controllable element (4, 5, 14, 17, 18, 21, 22, 23, 24) or each of the volume-controllable elements has an at least partially resilient outer wall.

6. The fluid pump according to claim 1 or one of the claims thereafter, **characterised in that** at least one blocking element (4, 5, 14, 17, 18, 21, 24) can be expanded in terms of its volume to a third volume, with which it fills part of the fluid space beyond the space taken up with the first and second volume.

7. The fluid pump according to claim 1 or one of the claims thereafter, **characterised in that** the first blocking element (14, 17, 18) is designed in such a way that, when transferring from the first set volume of the blocking element to the second set volume, the inflow channel/outflow channel is first blocked and then some of the fluid in the fluid space is displaced with further volume increase and the transition to the third set volume.

8. The fluid pump according to claim 6 or 7, **characterised in that** the blocking element (4, 5, 14, 17, 18, 21, 24) comprises different wall regions that can be expanded differently by differences of the material and/or the material thickness.

9. The fluid pump according to claim 7 or 8, **characterised in that** the blocking element (4, 5, 14, 17, 18, 21, 24), at least in a state in which it blocks the inflow channel to the greatest possible extent and still displaces a minimal volume of the fluid space, in particular also in the relaxed state, has a form that tapers in cross section toward the fluid space, wherein the broad base of the blocking element is arranged at the blockable inflow channel/outflow channel.

10. The fluid pump according to claim 1 or one of the claims thereafter, **characterised in that** the fluid space forms a connecting channel between the inflow channel and the outlet channel, and **in that** the conveying arrangement in the connecting channel comprises a displacing element (3, 22, 23) of which the volume can be controlled and which releases the connecting channel at least in portions with a first set volume and at least predominantly fills the connecting channel with a second set volume.

11. The fluid pump according to claim 10, **characterised in that** the conveying arrangement, in addition to the displacing element (3, 22, 23), comprises a blocking element (4, 5, 21, 24) both in the inflow channel and in the outflow channel.

12. The fluid pump according to claim 10 or 11, **characterised in that** the conveying arrangement comprises at least two, in particular three, four or five or more than five, individually controllable displacing elements (3, 22, 23).

13. The fluid pump according to claim 10, 11 or 12, **characterised in that** the displacing element(s) (3, 22, 23) is/are resiliently expandable in a balloon-like manner.

14. The fluid pump according to claim 13, **characterised in that** the displacing element(s) (3, 22, 23) is/are cylindrical or spherical.

15. The fluid pump according to claim 10 or one of the claims thereafter, **characterised in that** the blocking elements and the displacing elements (3, 22, 23) are connected controllably to a pressure source (52) via individual pressure lines (26, 27).

## Revendications

1. Pompe à fluide pour un premier fluide avec un compartiment à fluide, avec au moins un canal d'alimentation et au moins un canal d'évacuation pour le premier fluide, dans laquelle
a) le volume à remplir avec le premier fluide dans le compartiment à fluide peut être modifié par un changement de volume réglable d'un élément d'un dispositif de transport, dans lequel le dispositif de transport comporte au moins un élément de blocage (4, 5, 14, 17, 18, 21, 24) réglable quant à son volume, lequel libère au moins majoritairement, en particulier entièrement, un canal d'alimentation et/ou d'évacuation du compartiment à fluide en présence d'un premier volume réglé de l'élément de blocage, et bloque au moins majoritairement, en particulier entièrement, le canal correspondant en présence d'un deuxième volume réglé de l'élément de blocage (4, 5, 14, 17, 18, 21, 24), ou
b) la pompe à fluide est une pompe avec un rotor,
**caractérisée en ce que** le compartiment à fluide est entouré par un boîtier (43, 90) conçu de façon bistable et présentant un état de fonctionnement stabilisé expansé et au moins un autre état comprimé.

2. Pompe à fluide selon la revendication 1, **caractérisée en ce que** le boîtier (43, 90) comporte une région bombée (92) apte à être modifié entre un état bombé vers l'extérieur et un état bombé vers l'intérieur.

3. Pompe à fluide selon la revendication 2, **caractérisée en ce que** la région bombée (92) est partiellement cylindrique.

4. Pompe à fluide selon la revendication 1 ou l'une des suivantes, **caractérisée en ce que** l'élément/les éléments à volume réglable (4, 5, 14, 17, 18, 21, 22, 23, 24) comporte/comportent respectivement un compartiment à fluide à remplir avec un deuxième fluide.

5. Pompe à fluide selon la revendication 1 ou l'une des suivantes, **caractérisée en ce que** l'élément/les éléments à volume réglable (4, 5, 14, 17, 18, 21, 22, 23, 24) comporte/comportent respectivement une paroi extérieure au moins partiellement élastique.

6. Pompe à fluide selon la revendication 1 ou l'une des suivantes, **caractérisée en ce qu'**au moins un élément de blocage (4, 5, 14, 17, 18, 21, 24) peut être agrandi quant à son volume de manière à obtenir un troisième volume, dans lequel il remplit au moins une partie du compartiment à fluide au-delà de l'espace occupé par le premier et le deuxième volume.

7. Pompe à fluide selon la revendication 1 ou l'une des suivantes, **caractérisée en ce que** le premier élément de blocage (14, 17, 18) est aménagé de manière à ce que lors du passage du premier volume réglé de l'élément de blocage au deuxième volume réglé, le canal d'alimentation/d'évacuation est tout d'abord bloqué, puis une partie du volume dans le compartiment à fluide est refoulée, lors de l'augmentation supplémentaire du volume et du passage vers le troisième volume réglé.

8. Pompe à fluide selon la revendication 6 ou 7, **caractérisée en ce que** l'élément de blocage (4, 5, 14, 17, 18, 21, 24) comporte plusieurs régions de paroi susceptibles d'être expansées différemment par les différences de matériau et/ou l'épaisseur de matériau.

9. Pompe à fluide selon la revendication 7 ou 8, **caractérisée en ce que** l'élément de blocage (4, 5, 14, 17, 18, 21, 24) présente une forme affinée dans sa section transversale en direction du compartiment à fluide, au moins dans un état dans lequel il bloque quasiment entièrement le canal d'alimentation et refoule encore un volume minimal du compartiment à fluide, en particulier également dans un état détendu, dans laquelle la base large de l'élément de blocage est agencée sur le canal d'alimentation/d'évacuation pouvant être bloqué.

10. Pompe à fluide selon la revendication 1 ou l'une des suivantes, **caractérisée en ce que** le compartiment à fluide forme un canal de liaison entre le canal d'alimentation et le canal d'évacuation, et **en ce que** le dispositif de transport comporte un élément de refoulement (3, 22, 23) réglable quant à son volume dans le canal de liaison, lequel libère au moins par tronçons le canal de liaison en présence d'un premier volume réglé et le remplit au moins majoritairement en présence d'un deuxième volume réglé.

11. Pompe à fluide selon la revendication 10, **caractérisée en ce qu'**en plus de l'élément de refoulement (3, 22, 23), le dispositif de transport comporte un élément de blocage (4, 5, 21, 24) respectif aussi bien dans le canal d'alimentation que dans le canal d'évacuation.

12. Pompe à fluide selon la revendication 10 ou 11, **caractérisée en ce que** le dispositif de transport comporte au moins deux, en particulier trois, quatre ou cinq ou plus de cinq éléments de refoulement (3, 22, 23) réglables individuellement.

13. Pompe à fluide selon la revendication 10, 11 ou 12, **caractérisée en ce que** le(s) élément(s) de refoulement (3, 22, 23) est/sont expansible(s) élastiquement à la manière d'un ballon.

14. Pompe à fluide selon la revendication 13, **caractérisée en ce que** le(s) élément(s) de refoulement (3, 22, 23) est/sont cylindrique(s) ou sphérique(s).

15. Pompe à fluide selon la revendication 10 ou l'une des suivantes, **caractérisée en ce que** les éléments de blocage et les éléments de refoulement (3, 22, 23) sont reliés de façon réglable à une source de pression (52) par des conduits de pression individuels (26, 27).
